# EUROPEAN PATENT APPLICATION

(11) **EP 4 663 501 A1**
(43) Date of publication of application: **17.12.2025**
(21) Application number: 24181946.5
(22) Date of filing: 13.06.2024
(51) Int. Cl.: B60W 50/14, B60W 50/04, B60W 50/08, B60W 50/10, B60W 40/08, B60W 40/10

(54) **DRIVER ASSISTANCE SYSTEM**

(71) Applicant: Harman Becker Automotive Systems GmbH, 76307 Karlsbad (DE)
(72) Inventor: BANIK, Ananya, 76307 Karlsbad (DE)
(74) Representative: Westphal, Mussgnug & Partner, Patentanwälte mbB

(57) **Abstract**

Methods and system are herein provided for a driver assistance system. In one example, a driver assistance system for a vehicle comprises a computing device comprising one or more processors and a memory. The computing device is configured to receive first data, the first data comprising at least one of vehicle state data, environmental data, road data, map data, and occupant health data, perform predictive analysis on the first data, if, based on the predictive analysis of the first data, a problem is detected that is assumed to adversely affect safe driveability of the vehicle within a defined timeframe, determine whether the problem can be solved without interaction from an occupant of the vehicle, and if it is determined that the problem cannot be solved without interaction from an occupant of the vehicle, execute an emergency assistance application stored in the memory, wherein executing the emergency assistance application comprises interacting with an occupant of the vehicle via at least one user interface using generative artificial intelligence, Al, and emergency assistance application is executed until the problem is considered to be solved.

## Description

### TECHNICAL FIELD

Embodiments of the subject matter disclosed herein relate to driver assistance, and more particularly to personalized road safety assistance systems and methods.

### BACKGROUND AND SUMMARY

Driver assistance may include any type of relief that is provided to an individual associated with a vehicle to increase individual protection and enhance driver experience, with the aim of increasing driver safety and road safety. Such reliefs include a decreased stress level in the case of vehicle defects, health issues concerning the driver of the vehicle, and any other kind of emergency situations, or potential emergency situations. Driver assistance systems have been developed to assist the individual in controlling the vehicle, or at least subsystems of the vehicle. Car health monitoring systems, for example, output warnings to a driver of a vehicle if it is detected that car health may be impaired. Other advanced driver assistance systems (ADAS) can monitor a health status of the driver and/or the vehicle's environment and output a warning if any (potential) emergency situations are detected. There is, however, a risk that if a potential emergency situation is reported to a driver of a vehicle, the driver's stress level may rise steadily or even abruptly. This may negatively affect the driving behavior of the driver.

There is a need for a driver assistance system and related method that are able to reduce a driver's stress level during potentially hazardous situations, or even avoid a (significant) increase of a driver's stress level in the first place.

### SUMMARY

In one example, a driver assistance system for a vehicle comprises a computing device comprising one or more processors and a memory. The computing device is configured to receive first data, the first data comprising at least one of vehicle state data, environmental data, road data, map data, and occupant health data, perform predictive analysis on the first data, if, based on the predictive analysis of the first data, a problem is detected that is assumed to adversely affect safe driveability of the vehicle within a defined timeframe, determine whether the problem can be solved without interaction from an occupant of the vehicle, and if it is determined that the problem cannot be solved without interaction from an occupant of the vehicle, execute an emergency assistance application stored in the memory, wherein executing the emergency assistance application comprises interacting with an occupant of the vehicle via at least one user interface using generative artificial intelligence, AI, and emergency assistance application is executed until the problem is considered to be solved.

In another example, a method comprises receiving first data at a computing device of a driver assistance system of a vehicle, the first data comprising at least one of vehicle state data, environmental data, road data, map data, and occupant health data, performing, by means of the computing device, predictive analysis on the first data, if, based on the predictive analysis of the first data, a problem is detected that is assumed to adversely affect safe driveability of the vehicle within a defined timeframe, determining, by means of the computing device, whether the problem can be solved without interaction from an occupant of the vehicle, and if it is determined that the problem cannot be solved without interaction from an occupant of the vehicle, executing, by means of the computing device, an emergency assistance application stored in a memory of the computing device, wherein executing the emergency assistance application comprises interacting with an occupant of the vehicle via at least one user interface using generative artificial intelligence, AI, and emergency assistance application is executed until the problem is considered to be solved.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

FIG. 1 schematically illustrates a driver assistance system according to one or more embodiments of the present disclosure;
FIG. 2 schematically illustrates actions performed by a computing device of a driver assistance system according to one or more embodiments of the present disclosure; and
FIG. 3 shows a flowchart illustrating a method for a driver assistance system according to one or more embodiments of the present disclosure.

### DETAILED DESCRIPTION

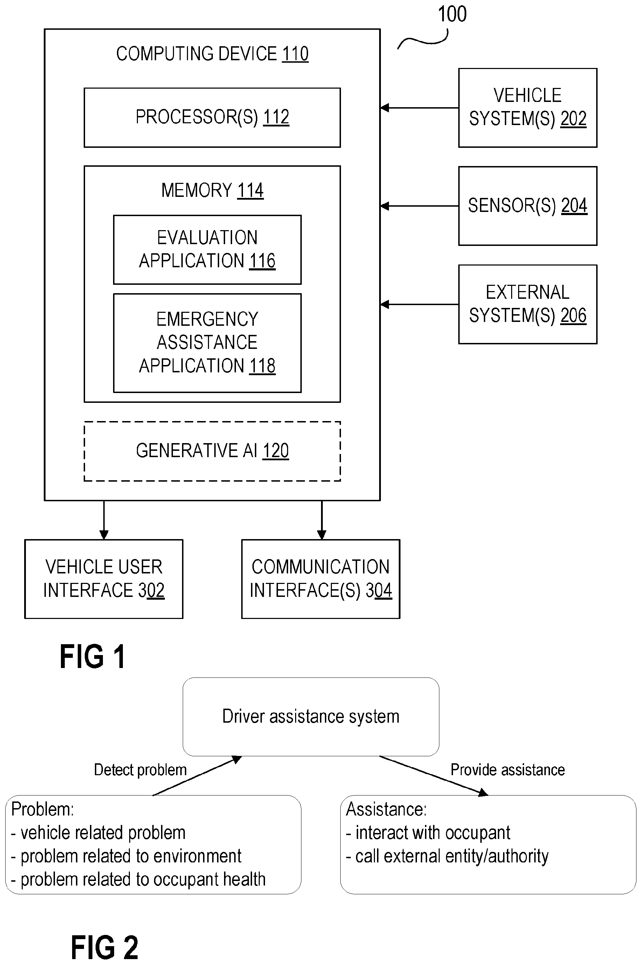

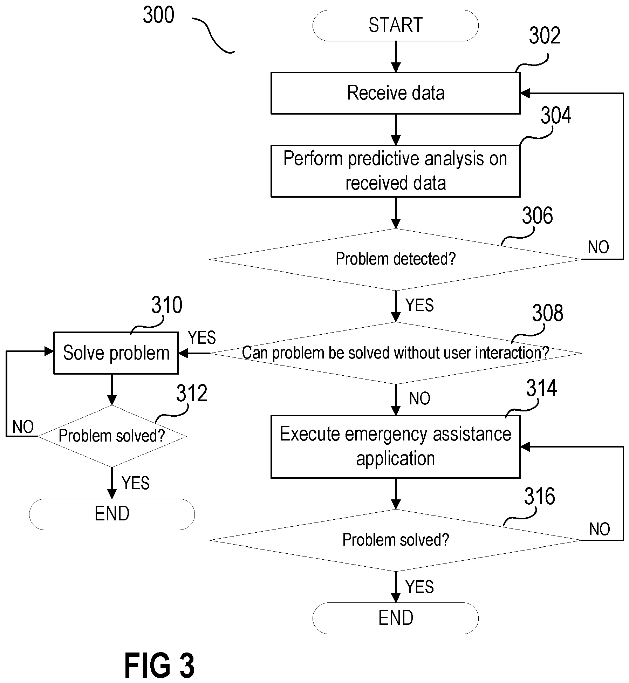

The following description relates to systems for a driver assistance system. The driver assistance system herein disclosed continuously monitors the vehicle and its components, the vehicle's surroundings, and/or one or more occupants of the vehicle and, if a problem is detected that is determined to require resolving, employs generative artificial intelligence, AI, to provide personalized assistance to the vehicle occupant(s) until the problem is considered to have been resolved. FIG. 1 shows a block diagram of a driver assistance system. FIG. 2 shows a data transmission pattern of the driver assistance system, and FIG. 3 shows a method for the driver assistance system.

Driving safety, among other factors, generally depends on a mental state and a mental stress level of the driver of a vehicle. Under mental stress, drivers are more likely to perform unexpected and/or hazardous driving maneuvers. A driver not focusing their entire attention to driving the vehicle may not be fully aware of their surroundings, e.g., of other road users or obstacles. This may put the driver and any passengers of the vehicle as well as other road users at risk, and may result in (serious) incidents. The driver assistance system and method thereof disclosed herein are able to provide guidance and support for occupants of a vehicle in order to solve any issues and problems emerging while driving the vehicle. In this way, occupants and especially the driver of the vehicle feel taken care of such that they are less likely to lose their focus on driving or even panic in emergency situations.

FIG. 1 schematically illustrates a driver assistance system 100 in a block diagram. The driver assistance system 100 for a vehicle comprises a computing device 110, the computing device 110 comprising one or more processors 112 and a memory 114. The computing device 110 is configured to receive first data, the first data comprising at least one of vehicle state data, environmental data, road data, map data, and occupant health data. The computing device 110 is configured to execute evaluation application 116 stored in the memory 114, and perform predictive analysis on the first data. Thus, the driver assistance system 100 considers data of the vehicle, its environment, the road the vehicle is traveling on, current map data, and/or health data of vehicle occupants to detect any problems concerning the vehicle, its surroundings or an occupant of the vehicle. The computing device 110 is further configured to, if, based on the predictive analysis of the first data, a problem is detected that is assumed to adversely affect safe drivability of the vehicle within a defined timeframe, determine whether the problem can be solved without interaction from an occupant of the vehicle. If, however, it is determined that the problem cannot be solved without interaction from an occupant of the vehicle, the computing device 110 is configured to execute an emergency assistance application 118 stored in the memory 114. Executing the emergency assistance application 118 comprises interacting with an occupant of the vehicle via at least one user interface 302 using generative artificial intelligence, AI, 120. That is, a two-way communication is performed, which allows the driver assistance system 100 to react to any input from the occupant(s) of the vehicle in real-time. The driver assistance system 100 is able to provide guidance for the driver or any passengers of the vehicle until the problem is considered solved, as execution of the emergency assistance application 118 is only terminated when the problem is considered solved.

Computing device 110 may be any kind of device that includes one or more processor(s) 112 such as a system-on-a-chip (SoC). In some embodiments, computing device 110 can be a head unit or other component included in a vehicle system. Generally, computing device 110 may be configured to coordinate the overall operation of driver assistance system 100. The embodiments disclosed herein contemplate any technically-feasible system configured to implement the functionality of driver assistance system 100 via computing device 110. In various embodiments, computing device 110 may be located in various environments including, without limitation, road and/or land vehicle environments such as, e.g., consumer vehicles, commercial vehicles, bicycles, motorcycles, wheeled drones, and so forth.

Processor(s) 112 may be any technically-feasible form of processing device configured to process data and execute program code. Processor(s) 112 could include, for example and without limitation, a system-on-chip (SoC), a central processing unit (CPU), a graphics processing unit (GPU), an application specific integrated circuit (ASIC), a digital signal processor (DSP), a field programmable gate array (PFGA), and/or the like. Processor(s) 112 may include one or more processing cores. In operation, processor(s) 112 may be a primary processor of the computing device 110, controlling and coordinating operations of other system components. For example, processor(s) 112 may be configured to execute instructions (e.g., methods, algorithms, processes, etc.) stored in memory 114.

Memory 114 stores evaluation application 116 and emergency assistance application 118, and may include a memory module or a collection of memory modules. Memory 114 may be non-transitory memory or other form of non-volatile memory, random access memory (RAM), or any other feasible type of memory storage system. In various embodiments, processor(s) 112 can execute evaluation application 116 to perform predictive analysis on the received first data, and execute instructions of the emergency assistance application 118 to implement the overall functionality of the computing device 110 and, thus, to coordinate the operation of the driver assistance system 100 as a whole. For example, first data received by the computing device 110 may be processed by the processor(s) 112 to determine whether execution of the emergency assistance application 118 is required. The computing device 110 may then execute the emergency assistance application 118. For example, processor(s) 112 may send one or more commands that initiate communication with an occupant of the vehicle via one or more user interfaces 302. In some embodiments, evaluation application 116 and emergency assistance application 118 may be stored and loaded into the memory 114 for execution.

The computing device 110, when determining whether a problem has occurred or is about to occur within a defined timeframe that is assumed to adversely affect safe driveability of the vehicle, may consider any data related to the vehicle itself, the vehicle's environment, the road the vehicle is driving on, map data, and/or health data of one or more occupants of the vehicle. Vehicle state data may comprise one or more of a current tire condition, age and wear of one or more vehicle components, vehicle dynamics, and operating state of one or more vehicle components, including one or more of vehicle engine and/or motor, powertrain, braking system, and suspension. The environmental data may comprise one or more of weather data, visibility data, and road condition data. Environmental data, however, can alternatively or additionally comprise data related to other road users within a defined radius from the vehicle, including, e.g., data concerning accidents or any other kind of hazardous events involving other road users. The road data can comprise one or more of road course, gradient of road, road surface quality, and road type. The map data can include one or more of road type, road course, and intersection data. Occupant health data can include one or more physiological parameters and one or more physical parameters of at least one occupant of the vehicle (e.g., of the driver of the vehicle and/or of one or more passengers of the vehicle). The one or more physiological parameters can include at least one of a heart rate, a respiratory rate, and a body temperature of the respective occupant, and the one or more physical parameters can include at least one of a size, a weight, an age, and a gender of the respective occupant.

In some vehicles, vehicle state data, environmental data, road data, map data, and occupant health data may be available for evaluation. Some vehicles monitor operating state of some or all vehicle components (e.g., high and medium class vehicles), while other vehicles (e.g., small or compact vehicles) do not provide such monitoring or only provide monitoring of some selected vehicle components. That is, depending on the kind of vehicle, vehicle state data may not be available or may only be available on a limited basis. Similarly, environmental data may only be fully available in some vehicles, e.g., in vehicles equipped with one or more cameras, or connected to one or more external systems 206. Road data is generally available if the vehicle comprises or is connected to a navigation system and/or comprises one or more cameras, for example. Map data is generally available if the vehicle comprises or is connected to a navigation system, for example. Occupant health data is available if the vehicle is equipped with one or more sensors 204 configured to monitor occupant health data and/or if computing device 110 is coupled to and receives data from external systems 206 such as, e.g., fitness tracking devices worn by an occupant of the vehicle. Computing device 100 evaluates any available first data. Overall function of the driver assistance system 100 is the same irrespective of what kind of data is available.

By performing predictive analysis on the first data (vehicle state data, environmental data, road data, map data and/or occupant health data), the driver assistance system is able to detect problems and predict any upcoming problems concerning the vehicle, its surrounding environment, and/or an occupant of the vehicle. Problems concerning the vehicle may include defects or failures of one or more vehicle components, for example, which may adversely affect safe driveability of the vehicle (e.g., failure of braking system). Problems concerning the environment of the vehicle may include accidents or hazardous events involving other road users that may adversely affect safe driveability of the vehicle. Problems concerning occupants of the vehicle may include any medical issues or emergencies that may adversely affect safe driveability of the vehicle (e.g., circulatory weakness of the driver of the vehicle).

For example, the computing device 110 may be coupled to and receive data from at least one of one or more vehicle systems 202, one or more sensors 204, and one or more external systems 206. The one or more vehicle systems 202 may include, for example, a navigation unit, a braking system, a tire monitoring system, a load sensing unit, a global positioning system (GPS), a steering input system, and the like. The one or more sensors 206 may include, for example, vehicle mounted sensors such as externally and/or internally facing cameras, rain sensors, thermometers, LiDAR sensors, RADAR sensors, ultrasonic and infrared sensors, weight sensors, thermal imaging sensors, and so on. Vehicles are often equipped with these sensors and vehicle systems as part of standard manufacturing. The computing device 110 may be configured to receive any data concerning the vehicle itself, its surrounding environment, and occupant health data from units mounted on or in the vehicle as well as from a cloud server, for example. Alternatively or additionally, the one or more sensors 206 may include, for example, wearable sensors that are configured to collect physiological and/or physical parameters of an occupant of the vehicle. Such sensors may include, e.g., wearable electrocardiogram, ECG, devices.

The computing device 110 may also be coupled to one or more external systems 206, such as servers or clouds. Alternatively or additionally, the computing device 110 may be configured for V2X communication, specifically vehicle-to-vehicle, V2V, communication, vehicle-to-pedestrian, V2P, and/or vehicle-to-infrastructure, V2I, communication. Thus, the computing device 110 may be configured to receive data regarding the environment, including road conditions, road data, and map data from external sources including other vehicles on the road and infrastructure including road side units (RSUs) and the like. Similarly, the computing device 110 may be configured to access the Internet or the like to obtain externally stored data such as weather data, including precipitation data, temperature data, and the like, traffic data, and more. Vehicles are also often equipped with one or more cameras. Information about a surrounding environment, including weather, road surface condition, road type, gradient, and status, traffic status information, and the like may be gathered by means of externally facing cameras. Information about a surrounding environment may additionally or alternatively be gathered by means of other sensors including rain sensors, thermometers, and the like as described above.

Vehicle state data received by the computing device 110, for example from the one or more vehicle systems 202, may include a current load of the vehicle, a current mark, type, and condition of tires of the vehicle, a type of the vehicle, vehicle dynamics, age, wear, and operation of one or more vehicle components, and current vehicle equipment information. The one or more vehicle components may include systems such as braking systems, suspensions, powertrains, engine and/or motor. Vehicle dynamics may include yaw rate, lateral acceleration, and steering input that affect vehicle handling. If, for example, the vehicle is comparably old, certain systems of the vehicle are more likely to fail as in comparably new vehicles. Similarly, the condition of many other vehicle components may be monitored. The current mark, type, and condition (e.g., wear, pressure, and temperature) of tires of the vehicle may also be monitored.

The current environmental data received by the computing device 110 may comprise at least one of current weather conditions in a surrounding environment of the vehicle, a visibility of the surrounding environment of the vehicle, a current road condition, and a current traffic volume. The current weather conditions may include parameters such as temperature, precipitation status, wind speed, and the like. As an example, upcoming heavy fog, rain or snow as well as black ice may be considered a problem that affects safe driveability of the vehicle and which cannot be solved without interaction from the driver of the vehicle.

The current road data received by the computing device 110 may comprise current road course of the road the vehicle is being driven on (e.g., straight, curvy, winding, etc.), and a current gradient of the road, a surface quality of the road (e.g., smooth, loose gravel, paved with potholes, paved without potholes, etc.). The current map data received by the computing device 110 may comprise type of road the vehicle is being driven on (e.g., multi-lane highway, single-lane street, residential street, forest road, etc.), a current road course, and information about intersections that are upcoming within a defined timeframe.

The data received by the computing device 110 is evaluated by means of evaluation application 116. Evaluation application 116 may use any feasible algorithm configured to perform predictive analysis and detect and/or predict problems that are assumed to adversely affect safe driveability of the vehicle within a defined timeframe (e.g., within the next several seconds or minutes, or during a current driving session). In one example, the evaluation application 116 may be configured to implement a monitoring digital twin system. A monitoring digital twin system allows constantly monitoring and performing predictive analysis on vehicle health. Monitoring digital twin systems, however, similarly allow constantly monitoring and performing predictive analysis on roadside emergency situations and/or occupant health.

A digital twin generally is an up-to-date representation of an actual physical asset in operation. As a representation of an asset in operation, a digital twin reflects the current asset condition and also includes relevant historical data about the asset. Digital twins can be used to evaluate the current condition of the asset and predict future behavior, refine the control, or optimize operation. The digital twin may include an artificial intelligence, AI, engine for performing predictive analysis on simulated data. All major parts of a vehicle can be monitored in this way (e.g., remotely in a backend server) while the vehicle's ignition is on. Different types of digital twins are generally known, e.g. dynamic digital twins and component-level twins, which may also interact with each other. The driver assistance system 100 may use any type of digital twin to perform predictive analysis. This allows to make predictions about any future problems related to the vehicle. The vehicle's environment and/or occupant health can be monitored in a similar way, in order to predict any problems in the vehicle's environment and/or concerning occupant's health. In some examples, the data may be collected locally within the vehicle. Over time, more and more data may be collected and analyzed by the driver assistance system 100. The driver assistance system 100 may thus take into account historic data as well as real time data when performing predictive analysis.

By using digital twin technology, the driver assistance system 100 is able to detect defects already at an early stage, e.g., before the defect actually arises. It further allows a very fast correction of any defects. The driver assistance system 100 is able to gather real time insights based on actual usage of the vehicle, and perform real-time problem diagnostics.

In this way, the driver assistance system 100 as herein presented, based on the available data concerning the vehicle, its surrounding environment, and one or more occupants of the vehicle, is able to detect problems related to the vehicle, the vehicle's environment, and/or an occupant of the vehicle that are assumed to adversely affect safe driveability of the vehicle within a defined timeframe (e.g., within the next several seconds or minutes, or during the current driving session). The driver assistance system 100 may determine whether a detected problem can be solved without requiring any interaction with an occupant of the vehicle. If it is determined that the problem cannot be solved without interaction from an occupant of the vehicle, emergency assistance application 118 stored in memory 114 is executed. As the driver assistance system 100 is arranged in the respective vehicle and comprises the computing device 110, the computing may be performed locally within the vehicle, e.g., via edge computing. Performing the evaluations, analysis and determinations locally may ensure a swift response and adaptation to changing conditions, especially as environmental conditions, road conditions, and occupant health conditions may change rapidly in some circumstances. The driver assistance system 100 thus provides a centralized system that aggregates data from various inputs for further evaluation, processing and analysis.

Some problems can be solved without requiring any interaction with an occupant of the vehicle. For example, some problems concerning the vehicle can be solved automatically by means of software updates or patch releases (e.g., firmware updates). Some problems concerning an environment of the vehicle can be solved, for example, by updating a route presented to the driver of the vehicle by means of a navigation system. That is, some problems concerning the environment of the vehicle may simply be bypassed. Any detected problems may be categorized accordingly, for example. Any problems that can be solved without requiring any interaction with an occupant of the vehicle can be categorized as solvable problems, for example, which do not require further attention from vehicle occupant(s).

If, however, it is determined that a problem cannot be solved without interaction from an occupant of the vehicle, emergency assistance application 118 stored in memory 114 is executed. Executing emergency assistance application 118 comprises interacting with an occupant of the vehicle via at least one user interface 302 using generative AI 120. Generative AI is configured to create images, text, videos, and other media in response to prompts from a user. That is, a 2-way communication with an occupant of the vehicle can be initiated by means of generative AI. During this communication, information concerning the detected problem can be communicated to the occupant. The driver assistance system 100 may further provide one or more potential solutions for solving the detected problem to the occupant. The occupant may enter prompts in order to agree or disagree with the proposed solution(s), and pose questions to the driver assistance system 100, for example. Some occupants may want to obtain more details concerning the detected problem, which may then be provided by the driver assistance system 100 as part of the communication with the occupant. The driver assistance system 100, by using generative AI 120, is able to seamlessly interact with the occupant, provide assistance as long as the problem exists, and provide personalized responses to any questions the occupant may have.

Executing the emergency assistance application 118 can further comprise establishing one or more connections to external entities and/or authorities via one or more communication interfaces 304. For example, the driver assistance system 100 may offer to initiate a call to an ambulance, the police or fire department, or even to a nearby garage. Upon approval from the occupant, the respective call may be initiated. The call may even be conducted by the driver assistance system 100. That is, the driver assistance system 100 may provide any necessary information concerning the detected problem to the respective entity or authority. Communication to external entities and/or authorities can also be carried out by means of generative AI 120. It is, however, also possible that the call is initiated by the driver assistance system 100, and the occupant themselves conduct the call and talk to the external entity or authority.

Communication with an occupant of the vehicle is conducted via one or more user interfaces 302. A vehicle may be equipped with a user interface 302, which may be a component of a dash or an infotainment unit in some examples. Information presented by the driver assistance system 100 may be outputted via the user interface 302 in an audio and/or visual manner. Input from an occupant may be received via the same or via a different user interface 302. According to some examples, driver assistance system 100 informs occupants of the vehicle about a detected problem by means of voice output. It is, however, also possible that, additionally or alternatively, occupants are informed about a detected problem by means of visual output, e.g., on a display of the vehicle. An occupant may input prompts by means of voice input, for example. Alternatively or additionally, occupant may input prompts or questions by operating a (touch-)display or pressing dedicated buttons, for example.

In this way, occupants are not only informed about an (upcoming) problem (e.g., by means of a suitable alert), but are able to gain a thorough understanding of the detected problem and any possible solutions for solving the problem. This makes the occupants feel safe and taken care of at all times such that they are less likely to lose their focus on driving or even panic in emergency situations. In many cases, problems can be predicted by the driver assistance system 100 before they actually occur (predictive analysis). In this way, appropriate measures can be already be taken before the problem actually occurs. An occupant, and especially the driver of a vehicle is generally even less likely to panic when informed of an upcoming problem in advance which does not yet adversely affect safe driveability of the vehicle, and when simultaneously presented with appropriate measures that can be taken to solve it. As the emergency assistance application 118 is executed until the problem is considered solved, occupants of the vehicle feel safe and secure at all times until the problem is considered solved. The driver assistance system 100 enables comprehensive risk management and provides support during any possible incidents and emergency situations. The driver assistance system communicates with occupants of a vehicle in an interactive way, as it does not only provide information about the detected problem and any possible solutions. As it uses generative AI in communicating with the occupant(s), it is able to seamlessly communicate with the occupant, respond to requests and questions an occupant may have, and assist the occupant in solving the problem.

Communicating with an occupant may even comprise presenting video content to the occupant(s). For example, one or more videos may be presented to the occupant(s) of the vehicle which visually demonstrate which actions may be taken by the occupant(s) themselves in order to solve the problem. If, for example, it is detected that a tire of the vehicle is losing pressure and the respective tire needs to be changed, a video may be presented to the occupant, illustrating the steps that are necessary in order to perform the wheel change. The occupant could be guided through the entire process, until the wire has been changed and the problem is considered solved. Instead of presenting a video, it is, however, also possible that the occupant(s) are guided through the process of solving the problem by means of audio output only. Alternatively, or additionally, it is also possible to present static images to an occupant, thereby illustrating any steps that are necessary for solving the problem. An occupant, when presented with such guidance, may carry out the required steps and solve the problem. It is, however, also possible that an occupant instead requests the driver assistance system 100 to find a nearby service station, or call a tow truck, for example. The driver assistance system 100 is able to respond to any preferences of an occupant.

Referring now to FIG. 2, exemplary interactions of the driver assistance system 100 according to various embodiments of the disclosure are schematically illustrated. The driver assistance system 100 analyses any available data and detects any problems related to the vehicle itself, the vehicle's environment, and/or an occupant's health that are assumed to adversely affect safe driveability of the vehicle within a defined timeframe. If it is determined that the problem cannot be solved without interaction from an occupant of the vehicle, assistance is provided to occupants of the vehicle. The driver assistance system 100 is able to interact with occupant(s) of the vehicle and communicate with external entities and/or authorities.

The driver assistance system 100 according to the various embodiments disclosed herein may be seamlessly integrated with already existing vehicle systems. The driver assistance system 100 may be implemented with only few additional components. The driver assistance system 100 may receive data from other systems and/or sensors already present in the vehicle, or from external systems and/or servers. The computing device 110 may analyze any such currently available data and predict any upcoming problems related to the vehicle, its environment, and/or the health of its occupants. The kind of data that is available to the driver assistance system 100 generally depends on the vehicle. Small and medium-sized cars may have less vehicle systems as compared to premium cars. For example, some vehicles do not comprise any sensors monitoring a state of the tires, brakes, or other vehicle systems. Other sensors such as, e.g., rain sensors, are usually also optional. Some vehicles do not have built-in navigation systems. The amount of data that is available, therefore, highly depends on the available sources. It is also possible that the driver assistance system 100 communicates with mobile devices such as smartphones, tablets, laptops, and so forth. The driver assistance system 100 may receive environmental data, road data or map data via such connection, for example.

Turning now to FIG. 3, a flowchart illustrating a method 300 for a driver assistance system of a vehicle, such as driver assistance system 100 of FIG. 1, is shown. The method 300 may be executed by one or more processors of the driver assistance system, such as processor(s) 112, based on instructions stored in memory, such as memory 114. The vehicle as herein described may be a gasoline or diesel powered vehicle, a battery electric vehicle, a hybrid electric vehicle, a plug-in hybrid electric vehicle, a hydrogen powered vehicle, or the like. The vehicle may be equipped with one or more sensors, including cameras, RADAR, GPS, and more, as described with respect to FIG. 1. The vehicle may include a plurality of vehicle systems, including braking systems, suspension, powertrains, an engine and/or electric motor, vehicle dynamics systems, and the like that are monitored at regular intervals. The vehicle may also be equipped with a V2X communication system configured for V2X communication with other vehicles, infrastructure, and a cloud, in some examples.

At 302, method 300 includes receiving first data. The first data includes at least one of vehicle state data, environmental data, road data, map data, and occupant health data. At 304 such data is evaluated by performing predictive analysis. If no problem is detected at 306, method 300 returns to step 302 and receives further, updated data, which is then again evaluated. If, at 306, a problem is detected that is assumed to adversely affect safe driveability of the vehicle within a defined timeframe, it is determined, at 308, whether the problem can be solved without interaction from an occupant of the vehicle.

If it is determined that the problem can be solved without interaction from an occupant of the vehicle, method proceeds to 310 and solves the problem, e.g., by means of a software update. In this case, method ends if the problem is considered to be solved at 312. If, however, at 308 it is determined that the problem cannot be solved without interaction from an occupant of the vehicle, emergency assistance application, such as emergency assistance application 118 of FIG. 1, is executed at 314. Executing the emergency assistance application includes interacting with an occupant of the vehicle via at least one user interface, using generative AI. According to various embodiments of the disclosure, interacting with the occupant of the vehicle using generative AI may comprise providing information concerning the detected problem to the occupant, providing one or more proposed solutions to solve the detected problem to the occupant, receiving input from the occupant, the input comprising prompts and/or questions, and responding to the input from the occupant. Emergency assistance application is executed as long as the problem is not considered solved at 316. If, at 316, the problem is considered solved, method 300 ends.

The method 300 may be performed whenever new data is available. For example, vehicle systems may provide updated vehicle state data at regular intervals. First data (i.e. vehicle state data, environmental data, road data, map data, and occupant health data) may be sent to the driver assistance system 100 automatically, or upon request. That is, driver assistance system 100 may send corresponding requests to vehicle system(s) 202, sensor(s), and/or external system(s) 206, e.g., at regular intervals and/or upon occurrence of a triggering event. According to some examples, method 300 may be performed at regular intervals, such as every 1 second, every 5 seconds, every 60 seconds, every 5 minutes, etc. Thus, any problems may be detected in real-time at each interval. A triggering event may include at least one of switch on of ignition, and a request received from an occupant of the vehicle, for example.

The following scenarios are provided to further exemplify the driver assistance system and methods as herein described, specifically to exemplify how the system may interact with an occupant and (optionally) also interact with external entities and/or authorities. Further, the following scenarios are presented as exemplary interactions with an occupant of a vehicle in situations where a problem has been detected. As a first non-limiting example, a failure of a vehicle system has occurred or will occur within a defined timeframe (e.g., within the next several seconds or minutes). The failure of the concerned system adversely affects driveability of the vehicle, but is not hazardous. Occupants of the vehicle are considered to be safe at all times. The vehicle's in-vehicle-infotainment system is not affected by the failure of the vehicle component. The driver assistance system starts execution of emergency assistance application, as the failure cannot be solved without interaction from vehicle occupants. Driver assistance system starts communicating with the occupant(s) of the vehicle. The volume of currently playing media is lowered, and a verbal description of the detected problem is output via vehicle loudspeakers. The driver assistance system asks occupant(s) of the vehicle, whether they desire to solve the problem themselves. If the occupant(s) wish for a detailed description how the problem can be solved, a respective description is provided by the driver assistance system, e.g., verbally or by means of images or videos. If the user does not wish to solve the problem themselves, the driver assistance system provides contact information for suitable service stations. The occupant(s) may either contact one or more service stations themselves, or may ask the driver assistance system to initiate and conduct respective calls. The driver assistance system may arrange a repair appointment for the occupant(s), for example. The driver assistance system is executed until the problem is considered solved. The problem may be considered solved when it has been fixed by the occupant(s), when a repair appointment has been fixed, or when the vehicle actually arrives at the service station, for example.

As a second non-limiting example, a driver of a vehicle is driving alone on a lonely road. An accident happens, in consequence of which the driver is injured and the vehicle is no longer driveable. The driver assistance system starts execution of emergency assistance application, as the problem cannot be solved without interaction from vehicle occupants. Driver assistance system starts communicating with the occupant(s) of the vehicle. If media is still playing, volume of currently playing media is lowered, and a verbal description of the detected problem is output via vehicle loudspeakers. Even further, possible solutions may be presented to the driver. The driver assistance system may enquire about the driver's condition, e.g., whether they feel capable of talking to external entities and/or authorities. The driver assistance system may initiate an emergency call and may conduct the call if the driver is not able or does not wish to conduct it themselves. The driver assistance system may, additionally or alternatively, contact an emergency contact number (previously) provided by the driver. For example, a close relative may be contacted and informed of the incident. If vehicle systems are not available after the accident, any calls may be initiated via the driver's phone, for example. The driver assistance system provides any required information to external entities and/or authorities such as, e.g., current location (e.g., GPS coordinates) of the vehicle, driver's condition, and so forth. The driver assistance system may continuously talk to the driver, or may talk to the driver regularly. For example, the driver assistance system may provide information to the driver that an ambulance has been requested and will arrive at the scene soon. The emergency assistance application is executed until the problem is considered solved. The problem may be considered solved, for example, when an ambulance, police, or relative of the driver arrives at the scene of the accident. The problem could also be considered solved when an unrelated third party arrives at the scene of the accident who stays with the driver until an ambulance arrives.

As a third non-limiting example, the driver assistance system may evaluate driver health data and, based on such data, determine that the driver of the vehicle is about to experience health issues (e.g., circulatory collapse). There is no other occupant in the vehicle other than the driver. The driver assistance system starts execution of emergency assistance application, as the problem cannot be solved without interaction from the vehicle driver. Driver assistance system starts communicating with the driver of the vehicle. If media is still playing, volume of currently playing media is lowered, and a verbal description of the detected problem is output via vehicle loudspeakers. The driver assistance system may recommend that the driver stop immediately or at the next best opportunity. Driver assistance system may request that the driver switch on hazard lights, or the driver assistance system may automatically switch on hazard lights. Driver assistance system may propose to the driver to drink water, if the driver has water available in the vehicle, and/or putting their feet up. Further, if it is determined that medical support is required, the driver assistance system may initiate an emergency call and may conduct the call if the driver is not able or does not wish to conduct it themselves. The driver assistance system may, additionally or alternatively, contact an emergency contact number (previously) provided by the driver. For example, a close relative may be contacted and informed of the incident. The driver assistance system may provide any required information to external entities and/or authorities such as, e.g., current location (e.g., GPS coordinates) of the vehicle, driver's condition, and so forth. The emergency assistance application is executed until the problem is considered solved. The driver assistance system may continuously talk to the driver, or may talk to the driver regularly. For example, the driver assistance system may provide information to the driver that an ambulance has been requested and will arrive at the scene soon. The problem may be considered solved, for example, when an ambulance, police, or relative of the driver arrives at the vehicle. The problem could also be considered solved when an unrelated third party arrives at the vehicle who stays with the driver until an ambulance arrives. The problem could also be considered solved when the driver of the vehicle has recovered and is considered fit to drive.

As a fourth non-limiting example, the driver assistance system may evaluate driver health data and, based on such data, determine that the driver of the vehicle is about to experience health issues (e.g., circulatory collapse). There is at least one other occupant present in the vehicle. The driver assistance system starts execution of emergency assistance application, as the problem cannot be solved without interaction from the vehicle driver. Driver assistance system starts communicating with the driver and passenger(s) of the vehicle. If media is still playing, volume of currently playing media is lowered, and a verbal description of the detected problem is output via vehicle loudspeakers. The driver assistance system may recommend that the driver stop immediately or at the next best opportunity. Driver assistance system may request that the driver or passenger(s) switch on hazard lights, or the driver assistance system may automatically switch on hazard lights. Driver assistance system may propose to the driver to drink water, if the driver has water available in the vehicle, and/or putting their feet up. If the medical issue is considered as non-critical, but the driver is not fit to drive, driver assistance system may propose that a passenger continue driving the vehicle, at least until the driver has recovered. If the driver's medical condition is considered to require medical attention, but is non-critical, driver assistance system may provide information concerning nearby doctors or hospitals. If it is determined that the driver's condition is critical, and immediate medical support is required, the driver assistance system may initiate an emergency call and may conduct the call if the driver and passenger(s) are not able or do not wish to conduct it themselves. The driver assistance system may, additionally or alternatively, contact an emergency contact number (previously) provided by the driver. For example, a close relative may be contacted and informed of the incident. The driver assistance system may provide any required information to external entities and/or authorities such as, e.g., current location (e.g., GPS coordinates) of the vehicle, driver's condition, and so forth. The emergency assistance application is executed until the problem is considered solved. The driver assistance system may continuously talk to the driver and passenger(s), or may talk to the driver and passenger(s) regularly. For example, the driver assistance system may provide information to the driver and passenger(s) that an ambulance has been requested and will arrive at the scene soon. The problem may be considered solved, for example, when an ambulance or police arrive at the vehicle. The problem could also be considered solved when the vehicle arrives at a doctor or hospital (vehicle driven by passenger). The problem could also be considered solved when the driver of the vehicle has recovered and is fit to drive.

In a fifth, non-limiting example, an incident may occur in proximity to the vehicle. For example, a bridge the driver intended to cross collapsed, a flooding of a street the driver intended to use occurred, or an attack happened at a nearby location. The driver may be alone in the vehicle or one or more passengers may be present. Occupants are considered safe at all times. The vehicle's in-vehicle-infotainment system is fully functional. The driver assistance system starts execution of emergency assistance application, as the failure cannot be solved without interaction from vehicle occupants. Driver assistance system starts communicating with the occupant(s) of the vehicle. The volume of currently playing media is lowered, and a verbal description of the detected problem is output via vehicle loudspeakers. The driver assistance system may initiate an emergency call. If the occupant(s) do not wish to conduct the call themselves, the driver assistant system may do so. Driver assistance system may provide any required information concerning the incident to the respective entity or authority. At the same time, driver assistance system may determine an alternative route on which the vehicle may continue its journey. The driver assistance system is executed until the problem is considered solved. The problem may be considered solved when it is confirmed by the external entity or authority that the vehicle may continue their journey, for example.

In the following, a non-limiting example of a possible communication between the driver assistance system, DAS, and a driver, Mr. Frank, of a vehicle is provided to further exemplify the driver assistance system and methods as herein described, specifically to exemplify how the system may interact with an occupant.
DAS: "Hello Mr. Frank, I detected a hole in the oil tank of your vehicle. The situation is not critical, but requires attention. According to my evaluation, you can still safely drive up to 40km.
I will assist you having it repaired in time."
Mr. Frank: "Hey DAS, what do you propose I should do now?"
DAS: "I found two service stations on your planned route. One of them can be reached without significant detour. I will add it as an additional destination on your active route. In the meantime, I am connecting to the service station to check their availability."
DAS: (initiates call to service station) "Hello, I am calling from Main Road and are 20km away from your service station. I have detected a hole in the oil tank of my vehicle which needs to be repaired. I will probably arrive at your service station in about 40 minutes. Will you be able to fix this immediately as a priority?"
Service station: "Yes we can handle the repair upon your arrival."
DAS: "Mr. Frank, the service station is able to fix the problem. The destination has been added to your active route. Please make sure to not drive faster than 40 km/h."

The emergency assistance system in this example may be executed until the vehicle arrives at the service station, and the problem is considered solved. The driver assistance system may alert the driver if they are exceeding a speed that is considered safe under the current circumstances, and may answer any questions the driver might have until the vehicle arrives at the service station. In this way, the driver feels safe and taken care of at all times until the problem is solved upon arrival at the service station.

The emergency assistance application of the driver assistance system herein provided gets activated by one or more of the following triggers: sensing a (probable) failure of a vehicle system; sensing a (probable) health issue of the driver or any passengers of the vehicle; sensing an emergency or incidence in the surrounding environment of the vehicle. According to further embodiments, the emergency assistance application of the driver assistance system may also gets activated in response to a user's request (e.g., by perceiving a keyword such as "Hey SystemName"). The technical effect of the methods and systems herein provided is that, as the driver assistance system described herein provides two-way interaction with an occupant of a vehicle when a problem concerning the vehicle, the vehicle's environment or an occupant of the vehicle is detected and which requires attention of an occupant of the vehicle, it is able to provide guidance and/or active support, thereby significantly reducing a mental load and mental stress of the occupant, and in particular of a driver of the vehicle. Different drivers and passengers of vehicles may react differently in identical situations. Some drivers may easily panic if a problem concerning the vehicle, the vehicle's environment or the health of an occupant of the vehicle is detected. This may negatively affect a driving behavior of the driver, and may even lead to (fatal) accidents. By interacting with the driver and/or other passenger(s) of the vehicle using generative AI until the problem is considered to be solved, the driver assistance system assists the driver and/or passenger(s) of the vehicle in acting in a controlled way and in staying calm even in emergency situations. The driver assistance system significantly improves road safety, as vehicle occupants, and especially the driver, are less likely to panic even in emergency situations.

The following claims particularly point out certain combinations and sub-combinations regarded as novel and non-obvious. These claims may refer to "an" element or "a first" element or the equivalent thereof. Such claims should be understood to include incorporation of one or more such elements, neither requiring nor excluding two or more such elements. Other combinations and sub-combinations of the disclosed features, functions, elements, and/or properties may be claimed through amendment of the present claims or through presentation of new claims in this or a related application. Such claims, whether broader, narrower, equal, or different in scope to the original claims, also are regarded as included within the subject matter of the present disclosure.

## Claims

1. A driver assistance system (100) for a vehicle, comprising:
a computing device (110) comprising one or more processors (112) and a memory (114), wherein the computing device (110) is configured to:
receive first data, the first data comprising at least one of vehicle state data, environmental data, road data, map data, and occupant health data;
perform predictive analysis on the first data;
if, based on the predictive analysis of the first data, a problem is detected that is assumed to adversely affect safe driveability of the vehicle within a defined timeframe, determine whether the problem can be solved without interaction from an occupant of the vehicle; and
if it is determined that the problem cannot be solved without interaction from an occupant of the vehicle, execute an emergency assistance application (118) stored in the memory (114), wherein
executing the emergency assistance application (118) comprises interacting with an occupant of the vehicle via at least one user interface (302) using generative artificial intelligence, AI, (120), and
emergency assistance application (118) is executed until the problem is considered to be solved.

2. The driver assistance system (100) of claim 1, wherein interacting with the occupant of the vehicle using generative AI comprises providing information concerning the detected problem to the occupant, providing one or more proposed solutions to solve the detected problem to the occupant, receiving input from the occupant, the input comprising prompts and/or questions, and responding to the input from the occupant.

3. The driver assistance system (100) of claim 1 or 2, wherein executing the emergency assistance application (118) further comprises establishing one or more connections to external entities and/or authorities via one or more communication interfaces (304).

4. The driver assistance system (100) of any of claims 1 to 3, wherein the vehicle state data comprises one or more of a current tire condition, age and wear of one or more vehicle components, vehicle dynamics, and operating state of one or more vehicle components, including one or more of vehicle engine and/or motor, powertrain, braking system, and suspension.

5. The driver assistance system (100) of any of claims 1 to 4, wherein performing predictive analysis on the first data comprises implementing one or more digital twins of the vehicle based on the vehicle state data.

6. The driver assistance system (100) of any of the preceding claims, wherein the environmental data comprises one or more of weather data, visibility data, road condition data, and data related to other road users within a defined radius from the vehicle.

7. The driver assistance system (100) of claim 6, wherein the data related to other road users comprises data concerning accidents or hazardous events involving other road users.

8. The driver assistance system (100) of any of the preceding claims, wherein the road data comprises one or more of road course, gradient of road, road surface quality, and road type.

9. The driver assistance system (100) of any of the preceding claims, wherein the map data comprises one or more of road type, road course, and intersection data.

10. The driver assistance system (100) of any of the preceding claims, wherein the occupant health data comprises one or more physiological parameters and one or more physical parameters of the driver of the vehicle, and/or one or more physiological parameters and one or more physical parameters of one or more passengers of the vehicle.

11. The driver assistance system (100) of claim 10, wherein
the one or more physiological parameters include at least one of a heart rate, a respiratory rate, and a body temperature of the respective occupant, and
the one or more physical parameters include at least one of a size, a weight, an age, and a gender of the respective occupant.

12. The driver assistance system (100) of claim 10 or 11, wherein the driver assistance system (100) is coupled to one or more sensors (204) arranged in the vehicle, the one or more sensors (204) being configured to collect the one or more physiological parameters and/or the one or more physical parameters.

13. The driver assistance system (100) of any of claims 10 to 12, wherein the driver assistance system (100) is coupled to one or more wearable sensors (204) and/or one or more external systems (206), the one or more external systems (206) comprising one or more of a fitness tracking device, a cloud or social network, the one or more wearable sensors (204) and/or one or more external systems (206) being configured to collect and/or store one or more physiological parameters and one or more physical parameters of one or more occupants of the vehicle.

14. A method, comprising:
receiving first data at a computing device (110) of a driver assistance system (100) of a vehicle, the first data comprising at least one of vehicle state data, environmental data, road data, map data, and occupant health data;
performing, by means of the computing device (110), predictive analysis on the first data;
if, based on the predictive analysis of the first data, a problem is detected that is assumed to adversely affect safe driveability of the vehicle within a defined timeframe, determining, by means of the computing device (110), whether the problem can be solved without interaction from an occupant of the vehicle; and
if it is determined that the problem cannot be solved without interaction from an occupant of the vehicle, executing, by means of the computing device (110), an emergency assistance application (118) stored in a memory (114) of the computing device (110), wherein
executing the emergency assistance application (118) comprises interacting with an occupant of the vehicle via at least one user interface (302) using generative artificial intelligence, AI, (120), and
emergency assistance application (118) is executed until the problem is considered to be solved.
